(19) Europäisches Patentamt
European Patent Office
Office européen des brevets

(11) **EP 4 316 362 A1**

(12) **EUROPEAN PATENT APPLICATION**
published in accordance with Art. 153(4) EPC

(43) Date of publication:
**07.02.2024 Bulletin 2024/06**

(21) Application number: **22775295.3**

(22) Date of filing: **15.03.2022**

(51) International Patent Classification (IPC):
**A61B 5/01** (2006.01)   **A61B 5/026** (2006.01)
**A61B 5/1459** (2006.01)   **A61B 5/37** (2021.01)
**A61B 5/372** (2021.01)

(52) Cooperative Patent Classification (CPC):
**A61B 5/01; A61B 5/026; A61B 5/1459; A61B 5/37; A61B 5/372**

(86) International application number:
**PCT/JP2022/011730**

(87) International publication number:
**WO 2022/202506 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.03.2021 JP 2021050393**

(71) Applicant: **Ant5 Co., Ltd.**
**Ube city, Yamaguchi, 755-0096 (JP)**

(72) Inventors:
• **SUZUKI Michiyasu**
**Ube-shi, Yamaguchi 755-8505 (JP)**
• **INOUE Takao**
**Ube-shi, Yamaguchi 755-8505 (JP)**
• **ARINDAM Gajendra Mahapatra**
**Ube-shi, Yamaguchi 755-8505 (JP)**

(74) Representative: **Hasegawa, Kan**
**Patentanwaltskanzlei Hasegawa**
**Untere Hauptstraße 56**
**85354 Freising (DE)**

(54) **CORTICAL SPREADING DEPOLARIZATION SENSING METHOD, SENSING SYSTEM, AND PROGRAM**

(57)   A cortical spreading depolarization sensing method includes: an acquisition step of acquiring, for each of a plurality of sites of the brain, waveform data indicating a temporal change in one or more types of biological information including at least any one of a direct current component of an electroencephalogram, hemoglobin concentration in the brain, cerebral blood flow, and brain temperature; a feature amount calculation step of determining windows set at predetermined time intervals on the waveform data and calculating a predetermined feature amount for each of the windows; an event extraction step of performing threshold processing on the feature amounts calculated in the feature amount calculation step to extract a timing of the occurrence of an event in which a value of the waveform data temporarily changes due to cortical spreading depolarization; a statistical processing step of acquiring the frequency distribution of the timings extracted based on the plurality of waveform data acquired in relation to a plurality of sites of the brain; and a determination step of determining whether cortical spreading depolarization has occurred, based on the frequency distribution.

**Fig.1**

```
          START
            │
   ┌──────────────────┐
   │     ACQUIRE      │── S10
   │  WAVEFORM DATA   │
   └──────────────────┘
            │
   ┌──────────────────┐
   │    CALCULATE     │── S11
   │ FEATURE AMOUNTS  │
   └──────────────────┘
            │
   ┌──────────────────┐
   │ EXTRACT TIMING OF│── S12
   │  THE OCCURRENCE  │
   │    OF EVENTS     │
   └──────────────────┘
            │
   ┌──────────────────┐
   │ ACQUIRE FREQUENCY│── S13
   │   DISTRIBUTION   │
   └──────────────────┘
            │
   ┌──────────────────┐
   │   DETERMINATION  │── S14
   └──────────────────┘
            │
           END
```

**Description**

TECHNICAL FIELD

The present invention relates to a method for sensing cortical spreading depolarization in a living brain, a sensing system and a program.

BACKGROUND ART

Conventionally, understanding the pathological condition of a patient takes place by analyzing biological information such as an electroencephalogram (EEG).

Patent Literature 1, for example, discloses a method of: acquiring EEG signals; extracting signal data of the bands of interest from the acquired EEG signals; obtaining a parameter of a nonlinear oscillator by minimizing an error function, which is a difference between the signal data value and the simulated value of the non-linear oscillator, in a window having a preset time width with respect to the signal data of the extracted band; and evaluating a characteristic of the EEG signal based on the property of the temporal change in the obtained parameter indicating the nonlinearity. In Patent Literature 1, the object of the aforementioned evaluation of EEG signals is for an epileptic condition.

Non-Patent Literature 1 discloses the use of a root mean square frequency and dominant frequency along with the ratio of their contributing parameter as feature amounts for classification of epileptic interictal and postictal electroencephalogram (EEG).

Non-Patent Literature 2 discloses a study relating to the effectiveness of EEG analysis in epileptic focus detection using entropy as a feature amount. In Non-Patent Literature 2, examples of entropies used in experiment of feature amount extraction include the Shannon entropy, Renyi entropy, generalized entropy, phase entropy, approximate entropy, sample entropy, and permutation entropy.

Non-Patent Literature 3 discloses the analysis of epileptic EEG data by means of using empirical permutation entropy as a feature amount.

Moreover, technologies of placing sensors intracranially to acquire biological information on the brain surface are also known. For example, the subdural sensors disclosed in Patent Literature 2 and Non-Patent Literature 4 can simultaneously measure cerebral blood flow, an EEG and brain temperature across a plurality of channels.

PRIOR ART DOCUMENTS

PATENT LITERATURE

Patent Literature 1: JP 2015-47452 A
Patent Literature 2: JP 6296606 B

NON-PATENT LITERATURE

Non-Patent Literature 1: Mahapatra et al., 'Classification of ictal and interictal EEG using RMS frequency, dominant frequency, root mean instantaneous frequency square and their parameters ratio', Biomedical Signal Processing and Control 44 (2018), pp. 168 to 180
Non-Patent Literature 2: SUGANO Hidenori et al., 'Focal diagnosis of epilepsy using entropy as a feature from interictal EEG', Japanese Journal of Clinical Neurophysiology 48(3), pp. 107 to 112, 2020
Non-Patent Literature 3: Unakafova, et al., 'Efficiently Measuring Complexity on the Basis of Real-World Data', Entropy 2013, 15, pp. 4392 to 4415
Non-Patent Literature 4: YAMAKAWA Toshitaka et al., 'Implantable Multi-Modality Probe for Subdural Simultaneous Measurement of Electrophysiology, Hemodynamics, and Temperature Distribution', IEEE TRANSACTIONS ON BIOMEDICAL ENGINEERING, VOL. 66, NO. 11, NOVEMBER 2019, pp. 3204 to 3211
Non-Patent Literature 5: SUGIMOTO Kazutaka et al., 'Spreading depolarization (CSD) appearing in subarachnoid hemorrhage - Blood flow is reduced when CSD reaches a pathological cerebral cortex', Japan Medical Journal, Weekly Nihon Iji Shimpo, No. 4791, 20 February 2016, p. 52

SUMMARY OF THE INVENTION

PROBLEMS TO BE SOLVED BY THE INVENTION

In a severe stroke or severe head trauma, for example, treating a disorder or controlling the preservation of brain function is very difficult because reduction of cerebrovascular autoregulation has a significant impact on changes in the intracranial physiological environment. Thus, the configuration of clinical indexes by placing sensors intracranially and extracting feature amounts from the various signals during the acute phase monitoring, are being investigated.

For example, when emergency surgery for a stroke takes place, a second surgery often takes place during an observation period of about two weeks. During this observation period, the physician normally comes to understand the changes of the pathological condition in the brain by observing an EEG as well as cerebral arterial blood pressure and intracranial pressure, and formulates a plan for drug administration treatment and a second surgery. However, understanding the changes of the pathological condition in the brain largely depends on the experience of the physician. Accordingly, a technology is desired to be able to quickly understand the condition in the brain on the basis of objective data such as the EEG, without being dependent on experience.

Cortical spreading depolarization (CSD) is known as an abnormal phenomenon occurring in the brain. CSD is a phenomenon where depolarization of neurons and glial cells in brain grey matter propagates like waves at a velocity of about 2 to 6 mm/min. It is understood that, in addition to exhibiting as a prodrome of a migraine headache, CSD also occurs in a cerebral infarction, cerebral hemorrhage, subarachnoid hemorrhage and head trauma (refer to Non-Patent Literature 5).

CSD comes with various changes in cerebral circulation and metabolism. Specifically, energy demand increases due to depolarization, as well as re-polarization which occurs in order to maintain the intracellular and extracellular ion gradient. Also, a variety of vasoactive factors such as potassium ions (K+) and nitrogen monoxide (NO) are released. If such CSDs reach a normal cerebral cortex, blood flow increases due to depolarization. On the other hand, if a CSD reaches a pathological cerebral cortex, the resistance blood vasoconstriction (referred to as a cerebral vasospasm) and cerebral blood flow is reduced. In other words, CSD can be said to be a phenomenon which appears preceding ischemia. Thus, if a CSD can be quickly sensed, the pathological condition of a patient can be predicted and treatment such as drugs can be administered beforehand, which can be useful in managing the prognosis.

CSD is observed as a so-called negative DC shift (hereinafter also simply referred to as DC shift), which is a phenomenon in which the direct current component (steady-state electric potential of the brain) of an EEG is temporarily reduced. However, it is difficult to quickly and precisely detect a DC shift which indicates the possibility of a CSD while simultaneously measuring EEG. Moreover, it is also difficult to determine whether a CSD occurred based on a detected DC shift because a DC shift is not necessarily observed only in conjunction with a CSD.

Taking into the consideration the aforementioned, an object of the present invention is to provide a cortical spreading depolarization sensing method which can quickly sense the possibility that a cortical spreading depolarization has occurred, a sensing system and a program.

MEANS FOR SOLVING THE PROBLEMS

In order to solve the aforementioned problem, a cortical spreading depolarization sensing method, which is one aspect of the present invention, includes: an acquisition step of acquiring, for each of a plurality of sites of a brain, waveform data indicating a temporal change in one or more types of biological information including at least any one of a direct current component of an electroencephalogram, hemoglobin concentration in the brain, cerebral blood flow, and brain temperature; a feature amount calculation step of setting windows set at predetermined time intervals on the waveform data and calculating a predetermined feature amount for each of the windows; an event extraction step of performing threshold processing on the feature amounts calculated in the feature amount calculation step to extract a timing of the occurrence of an event in which a value of the waveform data temporarily changes due to cortical spreading depolarization; a statistical processing step of acquiring a frequency distribution of the timings extracted based on the plurality of waveform data acquired in relation to a plurality of sites of the brain; and a determination step of determining whether cortical spreading depolarization has occurred based on the frequency distribution.

In the aforementioned cortical spreading depolarization sensing method, the determination step may include determining cortical spreading depolarization to have occurred when a peak value of frequency in the frequency distribution is in a predetermined range.

In the aforementioned cortical spreading depolarization sensing method, the determination step may include determining timings of peaks exceeding a first threshold in the frequency distribution except for timings of peaks exceeding a second threshold that is larger than the first threshold as timings of cortical spreading depolarization occurrence.

In the aforementioned cortical spreading depolarization sensing method, the acquisition step may include acquiring waveform data relating to a plurality of types of biological information; and the statistical processing step may include obtaining the frequency distribution based on the timings extracted based on the plurality of types of biological information.

In the aforementioned cortical spreading depolarization sensing method, the feature amount may be permutation entropy, and, the event extraction step may include extracting a negative peak below a threshold.

In the aforementioned cortical spreading depolarization sensing method, the feature amount may be power density, and, the event extraction step may include extracting a positive peak exceeding a threshold.

In the aforementioned cortical spreading depolarization sensing method, the windows may each have a width of longer than 0.5 minutes and shorter than 10 minutes.

In the aforementioned cortical spreading depolarization sensing method, the feature amount calculation step may include calculating the feature amount for data obtained by subjecting the waveform data to filtering process to cut off frequency components less than or equal to a predetermined frequency.

A sensing system, which is another aspect of the present invention, is provided with: a waveform data acquisition part configured to acquire, for each of a plurality of sites of a brain, waveform data indicating a temporal change in one or more types of biological information including at least any one of a direct current component of an electroencephalogram, hemoglobin concentration in the brain, cerebral blood flow, and brain temperature; a feature amount calculation part configured to set windows set at predetermined time intervals on the waveform data and calculate a predetermined feature amount for each of the windows; an event extraction part configured to perform threshold processing on the feature amounts calculated in the feature amount calculation part to extract a timing of the occurrence of an event in which a value of the waveform data temporarily changes due to cortical spreading depolarization; a statistical processing part to acquire a frequency distribution of the timings extracted based on the plurality of waveform data acquired in relation to a plurality of sites of the brain; and a determination part configured to determine whether cortical spreading depolarization has occurred based on the frequency distribution.

The aforementioned sensing system may be further provided with: a subdural sensor to be placed in the subdural space and capable of outputting signals indicating biological information concerning the brain; and a signal processing part capable of generating digital waveform data by subjecting signals output from the subdural sensor to a predetermined signal processing, where the waveform data acquisition part may acquire the waveform data from the signal processing part; and where the subdural sensor may have: a substrate formed from a flexible material and placed so as to be in contact with a brain surface, and a plurality of sensor units each mounted in a plurality of locations of the substrate on the side that is in contact with a brain surface, where each sensor unit may include at least any one of electrodes for measuring an electrocorticogram, a measurement part for measuring a hemoglobin concentration or a tissue oxygen saturation by near-infrared spectroscopy, a blood flowmeter for measuring a cerebral blood flow in accordance with the laser Doppler principle, and a temperature sensor.

A program, which is yet another aspect of the present invention, executes the following steps on a computer: an acquisition step of acquiring, for each of a plurality of sites of a brain, waveform data indicating a temporal change in one or more types of biological information including at least any one of a direct current component of an electroencephalogram, hemoglobin concentration in the brain, cerebral blood flow, and brain temperature; a feature amount calculation step of setting windows set at predetermined time intervals on the waveform data and calculating a predetermined feature amount for each of the windows; an event extraction step of performing threshold processing on the feature amounts calculated in the feature amount calculation step to extract a timing of the occurrence of an event in which a value of the waveform data temporarily changes due to cortical spreading depolarization; a statistical processing step of acquiring a frequency distribution of the timings extracted based on the plurality of waveform data acquired in relation to a plurality of sites of the brain; and a determination step of determining whether cortical spreading depolarization has occurred based on the frequency distribution.

According to the present invention, a feature amount is calculated on waveform data indicating a temporal change in biological information for each of the windows set, and threshold processing on this feature amount is performed to extract a timing of the occurrence of an event in which the value of the above waveform data temporarily changes due to cortical spreading depolarization. Thus, the possibility for that a cortical spreading depolarization has occurred can be quickly sensed.

BRIEF DESCRIPTION OF THE DRAWINGS

[Fig. 1] is a flowchart illustrating a cortical spreading depolarization sensing method according to an embodiment of the present invention.

[Fig. 2] is a block diagram illustrating one example of a sensing system in which the cortical spreading depolarization sensing method shown in Fig. 1 is executed.

[Fig. 3] is a schematic diagram illustrating one example of a subdural sensor which can be utilized in the embodiment of the present invention.

[Fig. 4] is a schematic diagram exemplifying a state in which the subdural sensor shown in Fig. 3 is placed in a subdural space.

[Fig. 5] is a graph illustrating a waveform of a direct current component of an electrocorticogram.

[Fig. 6] is a graph illustrating a waveform obtained by subjecting the waveform shown in Fig. 5 to high-pass filtering.

[Fig. 7] is a graph illustrating permutation entropy calculated based on the waveform shown in Fig. 6.

[Fig. 8] is a graph illustrating power density calculated based on the waveform shown in Fig. 6.

[Fig. 9] is a graph illustrating temporal modulation of amplitude calculated based on the waveform shown in Fig. 6.

[Fig. 10] is a graph illustrating temporal modulation of frequency calculated based on the waveform shown in Fig. 6.

[Fig. 11] is a graph illustrating temporal modulation of effective frequency calculated based on the waveform shown in Fig. 6.

[Fig. 12] is a graph illustrating sample entropy calculated based on the waveform shown in Fig. 6.

[Fig. 13] are graphs illustrating various types of feature amounts calculated based on the waveform shown in Fig. 5.

[Fig. 14] are graphs illustrating waveforms of direct current components of an electrocorticogram measured across six channels.

[Fig. 15] are graphs illustrating waveforms obtained by subjecting the waveforms shown in Fig. 14 to high-pass filtering.

[Fig. 16] are graphs illustrating feature amounts (permutation entropy) calculated based on the waveforms shown in Fig. 15.

[Fig. 17] are graphs illustrating the timings of events extracted based on the feature amounts shown in Fig. 16.

[Fig. 18] are graphs illustrating the timings of extracted events by a Raster display.

[Fig. 19] is a histogram illustrating a frequency distribution of the timing of events extracted in Channels 1 to 6.

[Fig. 20] is a smoothed graph of the histogram shown in Fig. 19.

[Fig. 21] is a graph illustrating the timings of CSD occurrences extracted from the graph shown in Fig. 20.

[Fig. 22] is a graph illustrating waveforms of brain temperatures, direct current components of electrocorticograms, and hemoglobin concentrations measured across three channels.

[Fig. 23] is a graph illustrating waveforms obtained by subjecting the waveforms shown in Fig. 22 to high-pass filtering.

[Fig. 24] is a graph illustrating the timings of events extracted based on the waveforms shown in Fig. 23, a frequency distribution of the extracted timings, and the timings of CSD occurrences determined based on the frequency distribution.

[Fig. 25] is an enlarged graph of the frequency distribution and timings of CSD occurrences shown in Fig. 24.

EMBODIMENTS OF THE INVENTION

A cortical spreading depolarization sensing method, a sensing system and a program according to an embodiment of the present invention are explained below, with reference to the drawings. However, the present invention is not limited by these embodiments. Moreover, the drawings are shown by appending the same reference numbers to the same parts thereto.

The drawings referred to in the below explanation merely schematically indicate the shapes, sizes and positional relationships to the extent that the content of the present invention may be understood. Namely, the present invention is not limited only to the shapes, sizes and positional relationships exemplified in the drawings. Moreover, there may include mutually differing sections of dimensional relationships and ratios among the drawings.

The sensing method of cortical spreading depolarization (CSD) explained below is executed on a computing device to quickly and automatically sense the possibility that a CSD has occurred.

A CSD is a phenomenon in which depolarization of neurons and glial cells in brain grey matter propagates like waves at a velocity of about 2 to 6 mm/min. Accordingly, if a CSD occurs, a phenomenon; that is, a so-called negative DC shift (hereinafter also simply referred to as DC shift), is observed in which a direct current component (DC component; namely a steady-state electric potential of the brain) is temporarily reduced in an electroencephalogram (EEG). However, a DC shift is not necessarily observed only in conjunction with a CSD, but can also occur, for example, due to factors such as a change in the contact condition of an electrode with the brain surface, or electrical or vibrational noise. Therefore, in order to accurately sense a CSD, it is critical to precisely detect an event such as DC shift, and to determine whether the detected event is caused by a CSD.

(Cortical spreading depolarization sensing method)

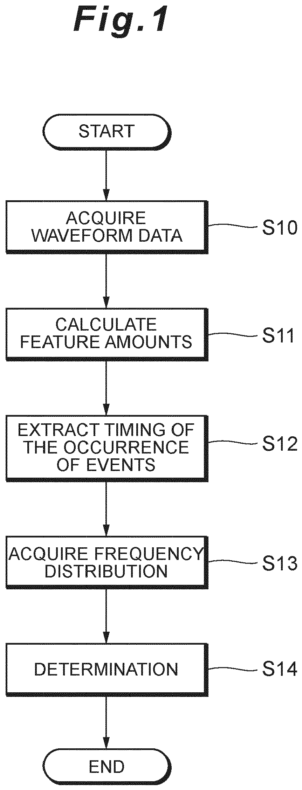

Fig. 1 is a flowchart illustrating a cortical spreading depolarization sensing method according to an embodiment of the present invention. The cortical spreading depolarization sensing method according to the present embodiment is executed schematically as follows, as shown in Fig. 1.

First, waveform data is acquired, where this data indicates a temporal change in one or more types of biological information including at least any one of a direct current component of an electroencephalogram (EEG), hemoglobin concentration in the brain, cerebral blood flow, and brain temperature (step S10).

Then, windows are set at predetermined time intervals on the acquired waveform data, and predetermined feature amounts are calculated for each of the windows (step S11). For example, permutation entropy and power density can be utilized as feature amounts.

Subsequently, threshold processing is performed on the calculated feature amounts to extract a timing of the occurrence of an event in which a value of the waveform data temporarily changes due to a CSD (step S12). For example, if the biological information is a direct current component of an EEG, the timing at which a steady-state electric current of the brain is temporarily reduced, namely, the timing at which a so-called negative DC shift occurred, is extracted as an event. The threshold processing is suitably set depending on the feature amount. As described in detail below, for example, if utilizing permutation entropy as a feature amount, it can be determined that an event occurred in a negative peak below a threshold. Moreover, if power density is utilized as a feature amount, it can be determined that an event occurred in a positive peak exceeding a threshold.

These steps S10 to S12 are executed for a plurality of waveform data based on biological information respectively measured in a plurality of sites of the brain.

Subsequently, a frequency distribution of the aforementioned timing of the occurrence of an event extracted in relation to a plurality of sites of the brain is acquired (step S13).

Then, it is determined whether a CSD occurred based on the frequency distribution of the aforementioned timing of the occurrence of an event in a plurality of sites of the brain (step S14). For example, if the peak value of frequency is in a predetermined range, it can be determined that a CSD occurred in the vicinity of the peak.

(Configuration example of a sensing system)

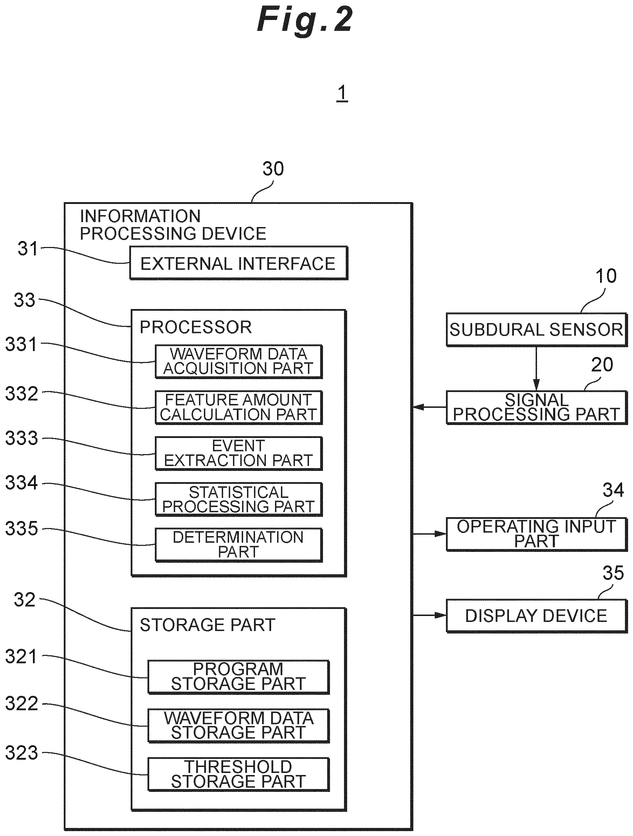

Fig. 2 is a block diagram illustrating one example of a sensing system in which the cortical spreading depolarization sensing method according to the embodiment of the present invention is executed. The sensing system 1 shown in Fig. 2 is provided with a subdural sensor 10, a signal processing part 20, and an information processing device 30. When detecting an event such as a negative DC shift in real time and quickly determining the possibility of a CSD while measuring the biological information such as the EEG of a person being examined, the system as exemplified in Fig. 2 in which a sensor and signal processing part are connected to the information processing device 30, can be utilized.

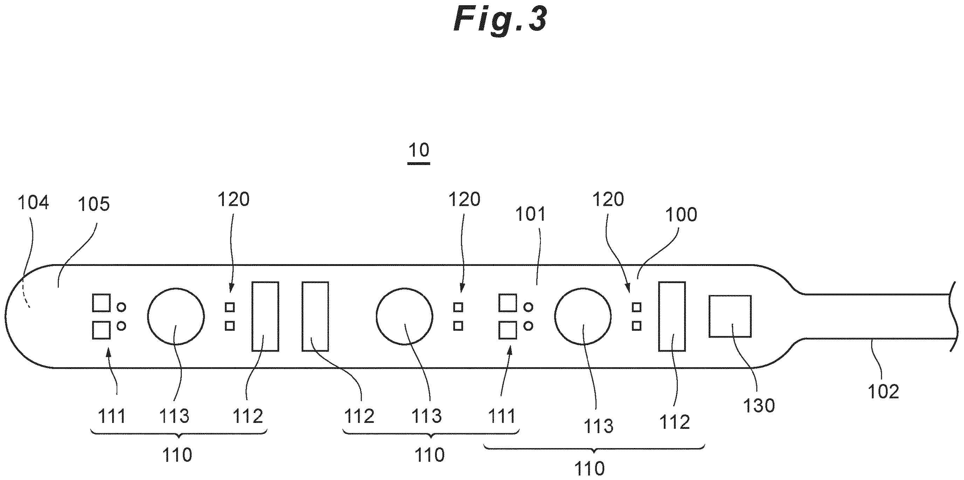

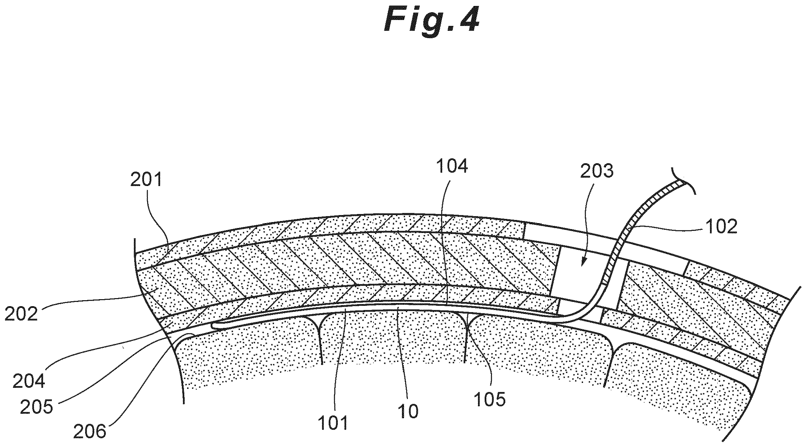

The subdural sensor 10 is a sensor which is placed in the subdural space and is capable of outputting signals indicating the biological information concerning the brain. Fig. 3 is a schematic diagram illustrating one example of a subdural sensor which can be utilized in the present embodiment. Fig. 4 is a schematic diagram exemplifying a state in which a subdural sensor is placed in the subdural space.

As shown in Fig. 3, the subdural sensor 10 is provided with a substrate 100 formed from a flexible material and placed so as to be in contact with a brain surface, and a plurality of sensor units 110 (three units in Fig. 3) which are mounted in the substrate 100 on the side that is in contact with a brain surface.

The substrate 100 is a so-called flexible substrate formed, e.g., by a resin material such as a polyimide. Except for the vertex vicinity of the electrode 113 described below, the substrate 100 and sensor unit 110 are integrally coated by a material having biocompatibility such as Parylene®.

The substrate 100 includes a sensor region 101 on which a plurality of sensor units 110 are placed, and a wiring region 102 continuous at one end side with a base end portion of sensor region 101. A connector for connecting this subdural sensor 10 to the signal processing part 20 is mounted at the other end side of wiring region 102. A wiring pattern connecting the sensor unit 110 and connector is formed at the sensor region 101 and wiring region 102.

Each sensor unit 110 includes an electrode 113 for measuring an electrocorticogram (EcoG). The electrode 113 is formed by, e.g., platinum, which obtains an electric potential of the brain surface.

Moreover, each sensor unit 110 may also include a blood flow measuring part, temperature measuring element (thermistor), intracranial pressure sensor, acceleration sensor, and a Doppler blood flow meter for measuring cerebral blood flow in accordance with the laser Doppler principle. For example, Fig. 3 shows a blood flow measurement part which includes light emitting elements 111 which can emit near-infrared light and light receiving elements 112 which can receive near-infrared light, and a temperature sensor 120. The blood flow measurement part measures a hemoglobin concentration or a tissue oxygen saturation, utilizing the near-infrared spectroscopy (NIRS) principle, where the cerebral blood flow condition can be estimated by means of the hemoglobin concentration or the tissue oxygen saturation. Moreover, an intracranial pressure sensor 130 may also be mounted to the sensor region 101.

The light emitting elements 111 radiate near-infrared light towards the brain. The light receiving elements 112 receive the near-infrared light reflected in the brain, and output the signals of this near-infrared light by converting these signals into electric signals. In the present embodiment, by placing the electrode 113 between the light emitting element 111 and light receiving element 112, the near-infrared light radiated from light emitting elements 111 and reflected in the brain is re-reflected by the electrode 113 towards the direction in the brain. Thereby, the near-infrared light reflected in the brain can be made to be efficiently incident onto the light receiving elements 112, and the sensitivity of signals in relation to the grey matter portion of the brain can be improved.

As shown in Fig. 4, when utilizing the subdural sensor 10, the scalp 201 is cut open and a burr hole 203 is formed in the cranium 202, and a smaller opening is further cut in the dura mater 204. From the gap of this dura mater 204, the sensor region 101 is inserted into the subdural space 205 such that the front surface 104 is on the dura mater 204 side, and the back surface 105 is on the brain surface 206 side. As shown in Fig. 3, because the sensor region 101 has a long and narrow shape, the sensor region 101 can be placed in the desired position without cutting a large opening into the cranium 202 and dura mater 24.

Because a plurality of sensor units 110 are placed on the sensor region 101, placing the subdural sensor 10 as illustrated in Fig. 4 can simultaneously acquire biological information in a plurality of sites of the brain. For example, the subdural sensor 10 shown in Fig. 3 can measure an electrocorticogram, cerebral blood flow (a hemoglobin concentration or a tissue oxygen saturation), as well as brain temperature, in three sites of the brain surface by means of the three sensor units 110. Of course, the sensor units 110 mountable to the subdural sensor 10 are not limited to three sensors, and may also be further increased.

Indeed, when measuring various kinds of biological information including an EEG, the subdural sensor 10 exemplified in Fig. 3 does not necessarily need to be used. For example, a sensor whose electrodes for measuring the EEG are placed in a matrix or concentric shape may also be used, and other known means may also be used.

Referring again to Fig. 2, the signal processing part 20 subjects the signals output from each channel of subdural sensor 10 to signal processing such as amplification and AD conversion, and inputs digital detection signals (waveform data) into the information processing device 30.

The information processing device 30 may be configured by means of a general purpose personal computer. The information processing device 30 is provided with an external interface 31, a processor 33 and a storage part 32. Moreover, an operation input part 34 and display device 35 may be provided in the information processing device 30.

The external interface 31 is an interface for connecting this information processing device 30 to external devices, as well as sending and receiving data to and from the external devices. In the present embodiment, the external interface 31 receives digital detection signals (waveform data) subjected to signal processing by the signal processing part 20.

The storage part 32 is configured by means of a computer readable storage medium, e.g., a semiconductor memory such as a ROM and RAM or a hard disk. The storage part 32 stores an operating system program, a driver program, application programs which execute various kinds of functions, as well as various kinds of parameters utilized during the execution of these programs, etc. In detail, the storage part 32 includes: a program storage part 321 which stores a program for sensing a brain condition indicating the possibility that a CSD has occurred; a waveform data storage part 322 which stores waveform data inputted from the signal processing part 20; and a threshold storage part 323 which stores thresholds utilized in the sensing of a brain condition.

The processor 33 is configured by means of, e.g., a CPU and reads various kinds of programs stored in storage part 32 to execute various kinds of processes for sensing a brain condition indicating the possibility that a CSD has occurred. Functional parts implemented by the processor 33 executing the programs stored in the storage part 32 include a waveform data acquisition part 331, a feature amount calculation part 332, an event extraction part 333, a statistical processing part 334, and a determination part 335.

The waveform data acquisition part 331 acquires digital detection signals (waveform data) detected by the subdural sensor 10 and subjected to signal processing by the signal processing part 20. Specifically, waveform data of one or more types of biological information including at least any one of a direct current component of an EEG, hemoglobin concentration in the brain, cerebral blood flow and brain temperature, is acquired.

The feature amount calculation part 332 sets windows set at predetermined time intervals on the waveform data acquired by the waveform data acquisition part 331, and calculates a predetermined feature amount for each of the windows.

The event extraction part 333 performs threshold processing on the feature amounts to extract timings of the occurrence of events in which a value of a waveform data temporarily changes due to a CSD.

The statistical processing part 334 acquires a frequency distribution of the aforementioned timings of the occurrence of events extracted in relation to a plurality of sites of the brain.

The determination part 335 determines whether a CSD occurred and outputs determination results, based on the frequency distribution of the timings of the occurrence of events.

The operation input part 34 is an input device which includes, e.g., an adjusting knob, a switch, a keyboard and a pointing device such as a mouse, where this operation input part 34 accepts input of commands and information into the information processing device 30.

The display device 35 is a display including, for example, a display panel formed by a liquid crystal or organic EL (electroluminescence) and a driving unit, which displays various kinds of waveform data, determination results of whether a CSD occurred etc., under the control of information processing device 30.

(Details of the cortical spreading depolarization sensing method)

The steps of the cortical spreading depolarization sensing method are explained in detail below by referring to the Examples. In the present example, direct current components of the electrocorticogram (ECoG) measured at six sites of the brain surface were used as biological information.

(First Example)

(1) Acquisition of waveform data

First, waveform data indicating a direct current component of an EEG is acquired. The direct current component can be acquired by subjecting detection signals of the EEG to low-pass filtering to cut off frequencies higher than 1 Hz.

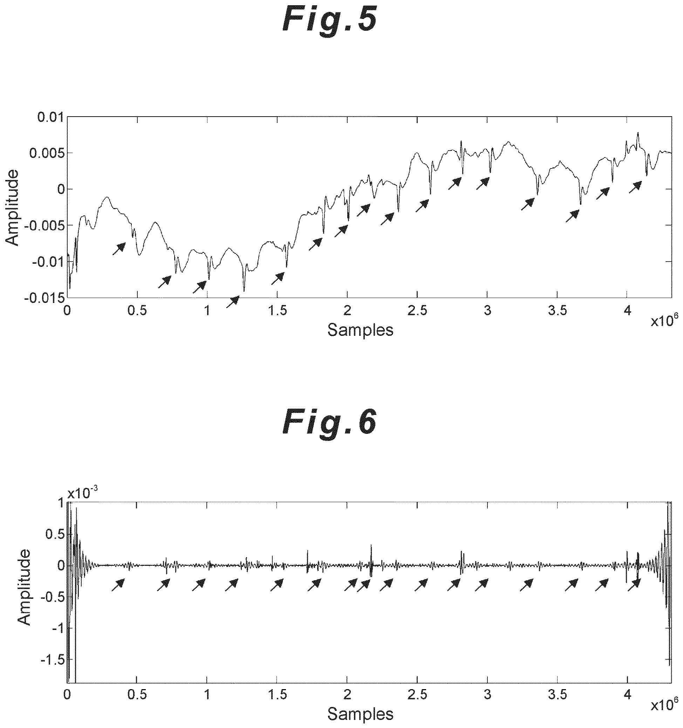

Fig. 5 is a graph illustrating a waveform of a direct current component of an electrocorticogram. In Fig. 5, the vertical axis indicates electrical voltage (mV). Moreover, the horizontal axis indicates the sample numbers of the time-sequentially acquired data, and corresponds to a time axis. The graph shown in Fig. 5, generated with a sampling frequency of 200 Hz, represents approximately six hours of data. The arrows shown in Fig. 5 indicate the timings of negative DC shift occurrences due to a CSD extracted manually by the present inventors.

(2) Calculation of the feature amount

Subsequently, a feature amount is calculated on the direct current component of the EEG for each of the windows set at predetermined time intervals. The windows preferably each have a width of longer than 0.5 minutes and shorter than 10 minutes, and more preferably 1 minute or more and 5 minutes or less. Moreover, the windows may also be set so that a portion thereof overlaps with adjacent windows.
In this case, high-pass filtering at cutoff frequency in the vicinity of 0.001 Hz to 0.0025 Hz may also be applied to the waveform data included in each of the windows. Here, a high-pass filtering is performed because, with the direct current component of the EEG as-is, great fluctuations in the baseline and large variations of 100 mV or more of the electric potential can also occur, and thus in order to remove signals considered to be of low neurophysiological significance beforehand. The high-pass filtering may be performed for each of the windows, or may also be performed on the entire waveform data. Moreover, the low-pass filtering for extracting the direct current components from the EEG waveform data, and the high-pass filtering for keeping an envelope constant may be performed simultaneously.
Fig. 6 is a graph illustrating a waveform obtained by subjecting the waveform shown in Fig. 5 to high-pass filtering to cut off frequency components of 0.0025 Hz or less. The arrows shown in Fig. 6 indicate the timings of negative DC shift occurrences, similarly to that of Fig. 5.
Permutation entropy or power density are preferably utilized as feature amounts.
Here, permutation entropy is a parameter expressing a time-sequential complexity, based on a comparison of adjacent values (refer to: Bandt, et al. 'Permutation Entropy: A Natural Complexity Measure for Time Series', PHYSICAL REVIEW LETTERS, VOLUME 88, NUMBER 17, 29 APRIL 2002, P. 174102-1 to P. 174102-4).
The permutation entropy can be obtained with the following Formula (1), by applying the existence probability $p(\pi)$ of a variety of permutations, to the Shannon entropy.
[Formula 1]

$$H(n) = -\sum_{\pi=1}^{n!} p(\pi) \log p(\pi) \quad \ldots (1)$$

In Formula (1), the symbol $\pi$ expresses types of permutations ($n \geq 2$) of the n-order. For example, if n = 2, the types of permutations in two time-sequentially adjacent data values $\{x_1, x_2\}$ are two types of increases ($x_1 < x_2$) and decreases ($x_1 > x_2$). Moreover, when n = 3, the types of permutations in three time-sequentially adjacent data values $\{x_1, x_2, x_3\}$ are six types of $x_1 < x_2 < x_3$; $x_1 < x_2 > x_3$ (where $x_1 < x_3$); $x_1 < x_2 > x_3$ (where $x_1 > x_3$); $x_1 > x_2 < x_3$ (where $x_1 < x_3$); $x_1 > x_2 < x_3$ (where $x_1 > x_3$); and $x_1 > x_2 > x_3$. Accordingly, $\pi$ can take on n! types of permutations.
Moreover, the symbol $p(\pi)$ is the probability that each of $\pi$ types of permutations exists in a time-sequential

data $\{x_t\}_t = _{1,\ldots,T}$, which can be conceptually expressed in the following Formula (2).
[Formula 2]

$$p(\pi) = \frac{\text{(NUMBER OF EACH TYPE OF PERMUTATIONS IN EXISTENCE)}}{T - n + 1} \quad \ldots(2)$$

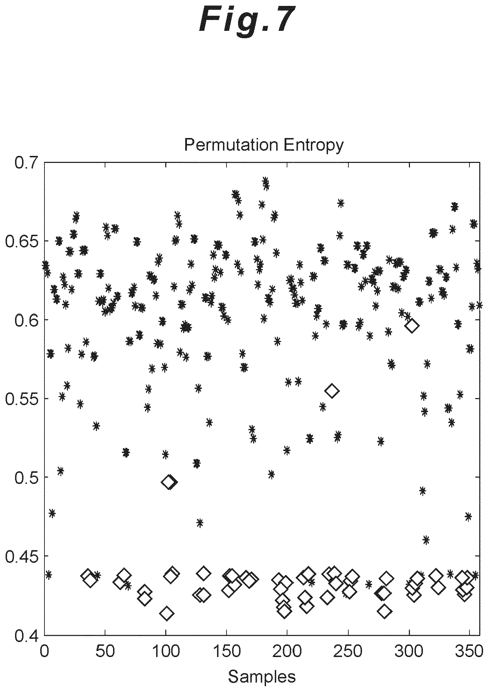

Fig. 7 is a graph illustrating permutation entropy calculated based on the waveform shown in Fig. 6. In Fig. 7, the vertical axis indicates the value of the permutation entropy, and the horizontal axis indicates time-sequential sample numbers, and corresponds to a time axis. In Fig. 7, the marks '0' indicate data points that correspond to the timings of negative DC shift occurrences due to a CSD extracted manually by the present inventors (refer to the arrows of Fig. 5). As shown in Fig. 7, most values of the permutation entropy when a negative DC shift occurs are concentrated in the lower values compared to values of the permutation entropy when a negative DC shift has not occurred. Accordingly, it is understood that when utilizing permutation entropy as a feature amount, data corresponding to a negative DC shift can be easily separated and extracted from other data.

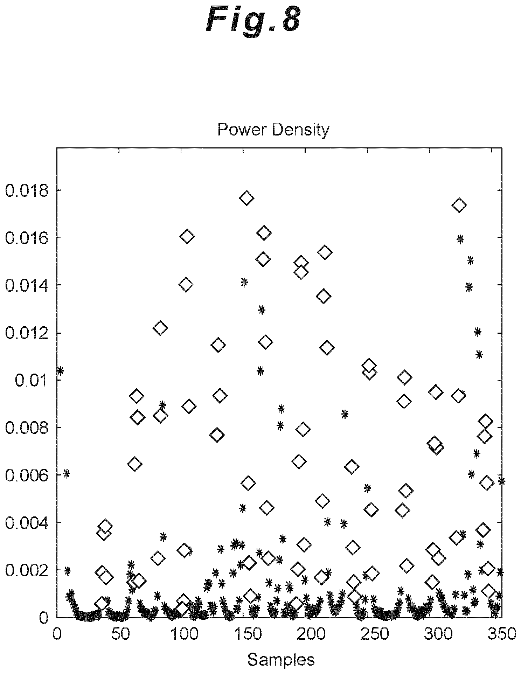

Moreover, power density can also be used as a feature amount. Power density is a time average value of a squared amplitude. Fig. 8 is a graph illustrating power density calculated based on the waveform shown in Fig. 6. In Fig. 8, the vertical axis indicates the value of the power density, and the horizontal axis indicates time-sequential sample numbers. Also in Fig. 8, the marks '0' indicate data points that correspond to the timings of negative DC shift occurrences due to a CSD extracted manually by the present inventors. As shown in Fig. 8, the power density when a negative DC shift has not occurred is substantially concentrated in the lower values. In contrast, the power density when a negative DC shift has occurred is distributed in the higher values to a certain extent. Accordingly, it is understood that when also using the power density as a feature amount, data corresponding to the negative DC shift can be easily separated and extracted from other data.

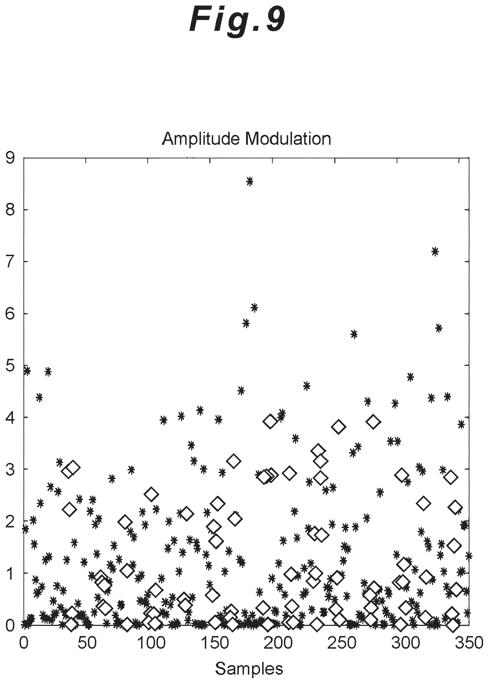

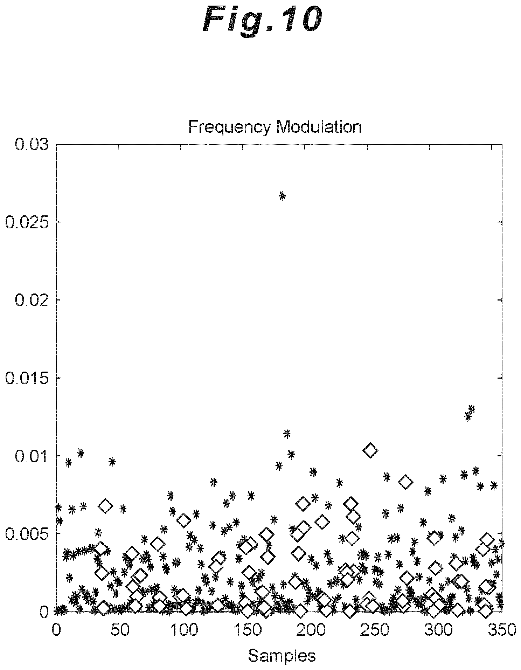

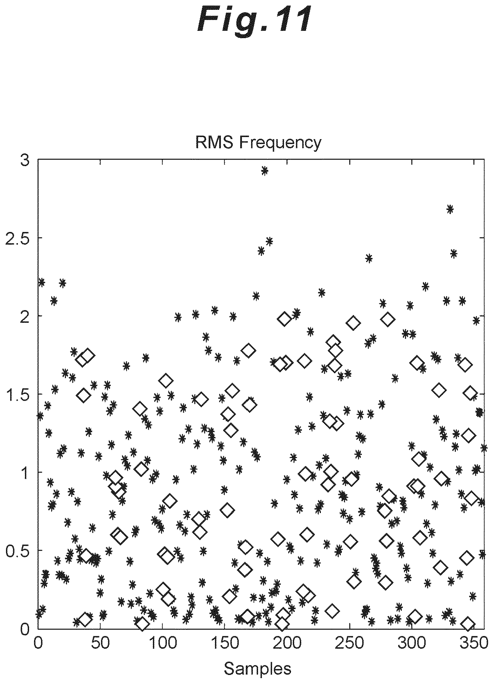

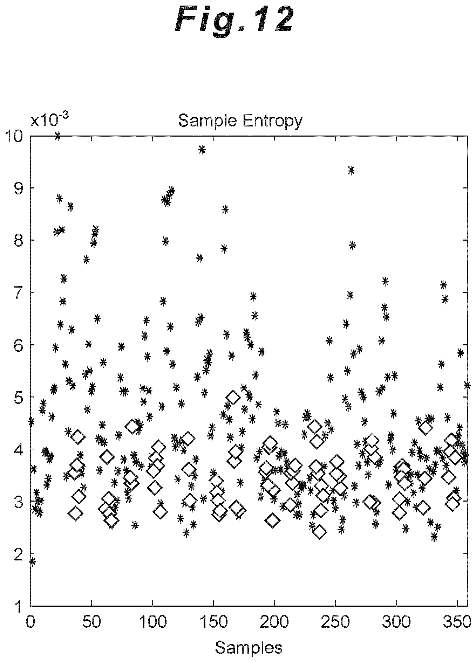

Examples of other calculated feature amounts are illustrated in Figs. 9 to 12 as comparative examples. Fig. 9 is a graph illustrating temporal modulation of amplitude calculated based on the waveform shown in Fig. 6. Fig. 10 is a graph illustrating temporal modulation of frequency calculated based on the waveform shown in Fig. 6. Fig. 11 is a graph illustrating temporal modulation of effective frequency calculated based on the waveform shown in Fig. 6. Fig. 12 is a graph illustrating sample entropy calculated based on the waveform shown in Fig. 6. In Fig. 9 to Fig. 12, the vertical axis indicates the values of each feature amount. The horizontal axis indicates time-sequential sample numbers, and corresponds to the time axis. Moreover, similarly to that of Fig. 7 and Fig. 8, the marks '0' indicates data points that correspond to the timings of negative DC shift occurrences due to a CSD.

Also in any of Figs. 9 to 12, data corresponding to the negative DC shift is mixed with other data. Therefore, it is understood that when using amplitude, frequency, effective frequency and sample entropy as feature amounts, it is difficult to separate and extract the data corresponding to the negative DC shift from the other data.

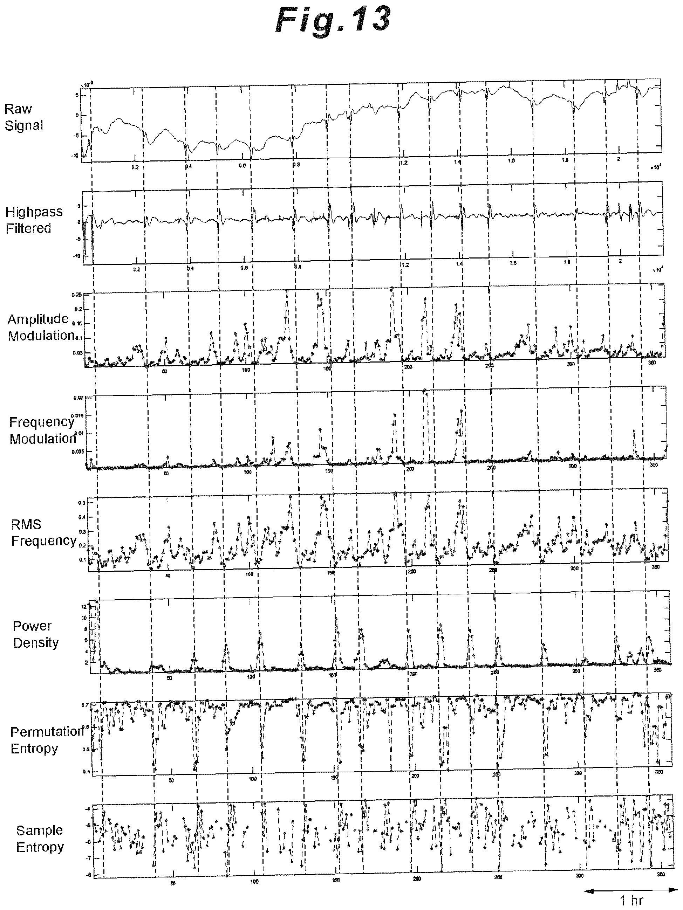

Fig. 13 are graphs illustrating various types of feature amounts calculated based on the waveform shown in Fig. 5. The dashed-dotted lines shown in Fig. 13 indicate the timings of negative DC shift occurrences due to a CSD extracted manually by the present inventors (refer to the arrows of Fig. 5 and Fig. 6). As shown in Fig. 13, the negative peaks (valleys) or positive peaks in the permutation entropy and power density match well with the timings of negative DC shift occurrences. In contrast, in the amplitude (Amplitude Modulation), frequency (Frequency Modulation), effective frequency (RMS Frequency) and sample entropy (Sample Entropy), no significant correlation can be seen between the changes in the various types of feature amounts and the timings of negative DC shift occurrences. It is also understood from these graphs that it is effective to use the permutation entropy and power density as feature amounts in the detection of the negative DC shift.

(3) Extraction of a timing of the occurrence of an event

Subsequently, by performing threshold processing on the feature amounts calculated, the timings in which a negative DC shift occurred are extracted as events due to a CSD. The threshold processing is suitably set depending on the feature amounts.

For example, if the feature amount is permutation entropy, the negative peaks (valleys) in the permutation entropy match well with the timings of negative DC shift occurrences, as shown in Fig. 13. Therefore, a negative peak below a threshold can be extracted as the timing of the occurrence of an event. Alternatively, as shown in Fig. 7, values of the permutation entropy in the timings of negative DC shift occurrences have significantly small values in contrast to that of the permutation entropy in other timings. Accordingly, the timings of which the permutation entropy is a threshold Th or lower may also be extracted.

Moreover, if the feature amount is power density, the positive peaks in the power density match well with the timings of negative DC shift occurrences, as shown in Fig. 13. Therefore, a positive peak exceeding a threshold can be extracted as the timing of the occurrence of an event. Alternatively, as shown in Fig. 8, values of the power density in the timings of negative DC shift occurrences have significantly large values in contrast to that of the power density in other timings. Accordingly, the timings of which the permutation entropy is a threshold Th or higher may be extracted.

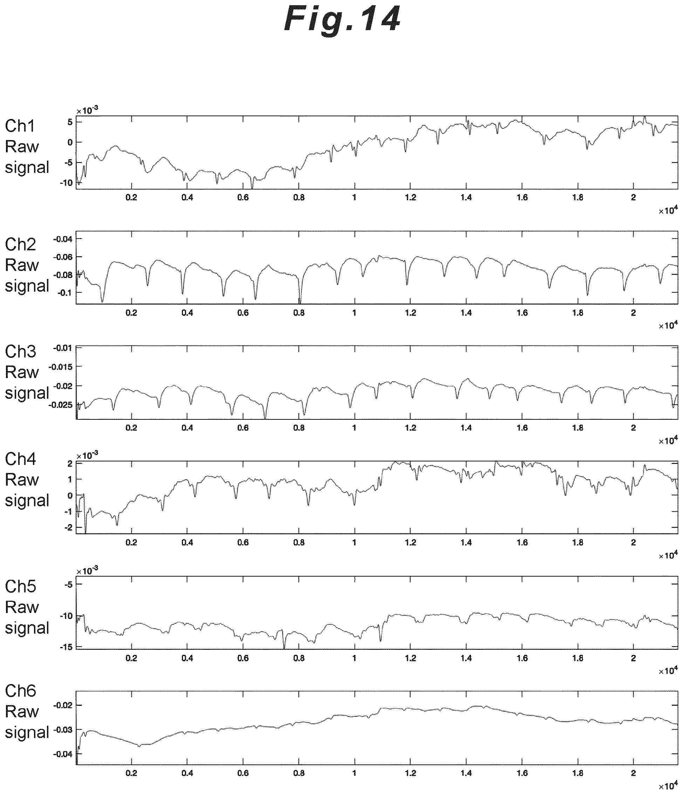

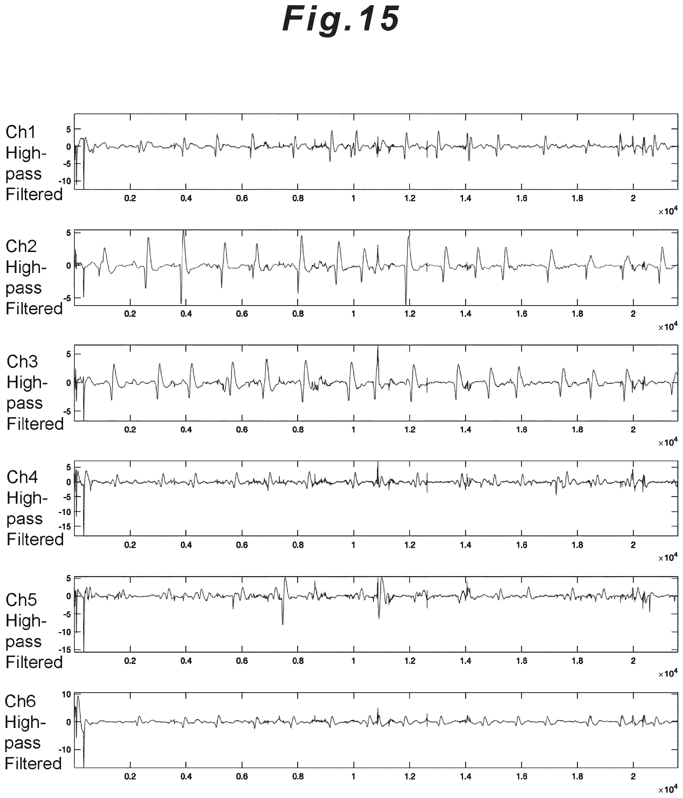

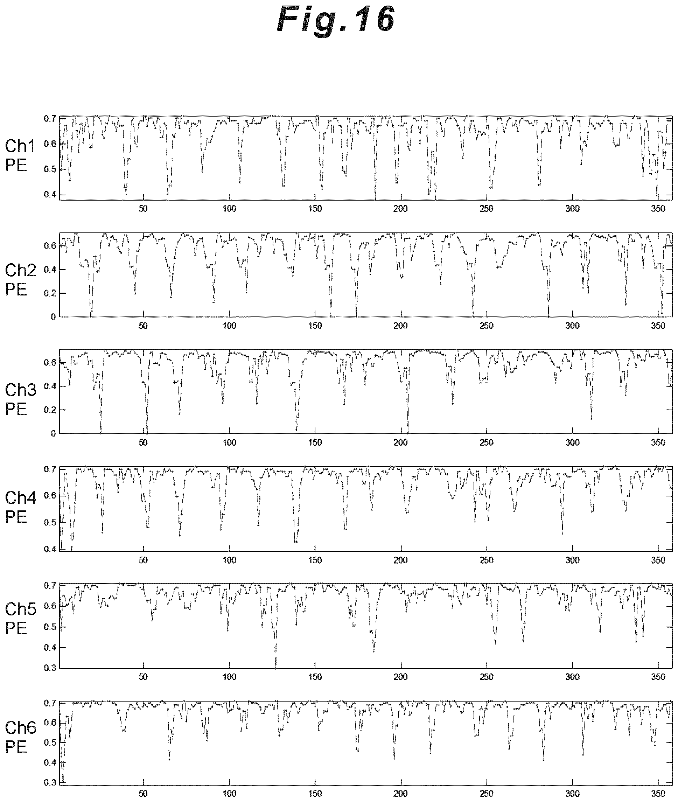

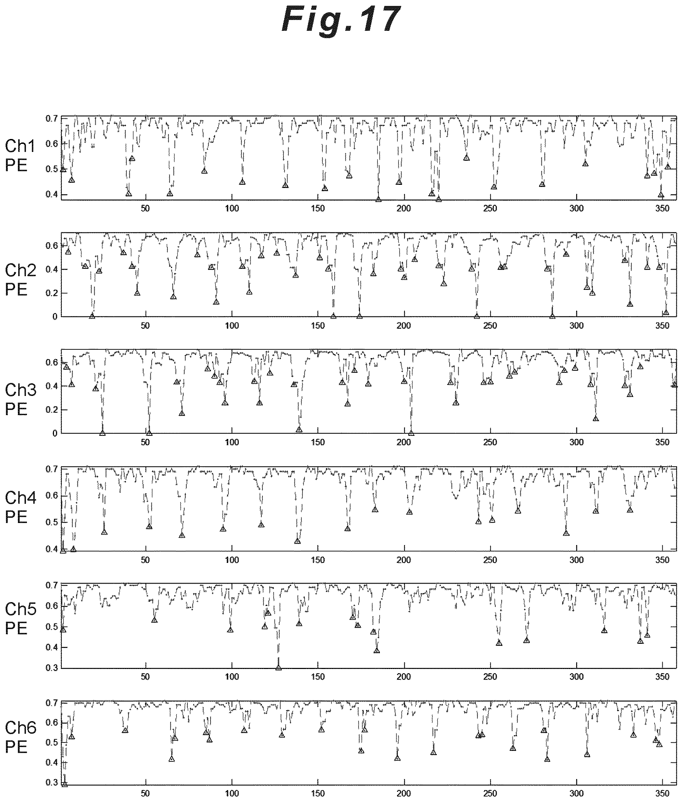

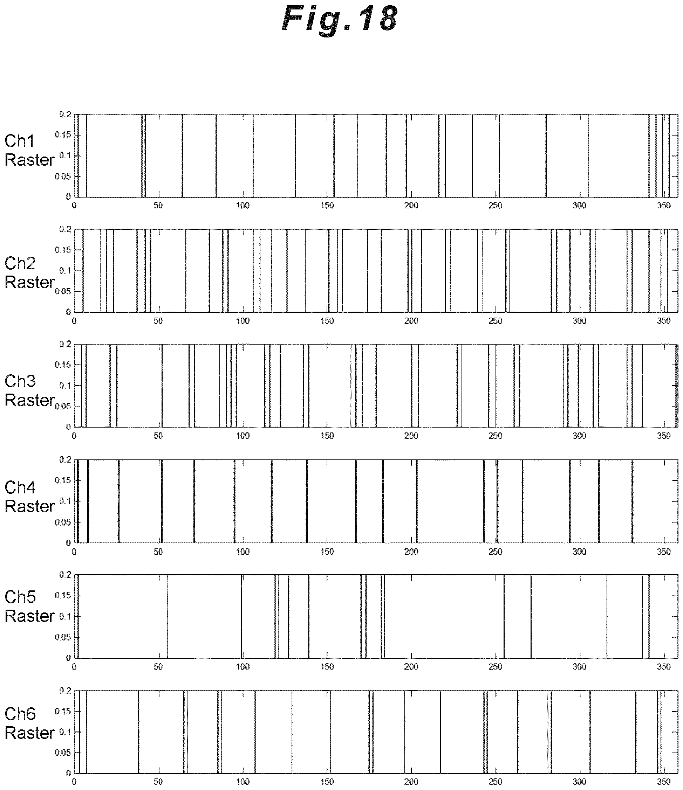

Fig. 14 are graphs illustrating waveforms of direct current components of an electrocorticogram measured across six channels. Fig. 15 are graphs illustrating waveforms obtained by subjecting the waveforms shown in Fig. 14 to high-pass filtering at a cutoff frequency of 0.0025 Hz. Fig. 16 are graphs illustrating feature amounts (permutation entropy) calculated based on the waveforms shown in Fig. 15. Fig. 17 are graphs illustrating the timings of events (negative DC shifts) extracted based on the feature amounts shown in Fig. 16. Fig. 18 are graphs illustrating the timings of extracted events by a Raster display. In Figs. 16 to 18, the horizontal axis indicates data numbers of feature amounts calculated by setting windows in the waveforms, and corresponds to the time axis. In Figs. 16 and 17, the vertical axis indicates the values of the feature amounts. In Fig. 17, the points indicated with the mark '△' are points extracted as events.

(4) Statistical processing

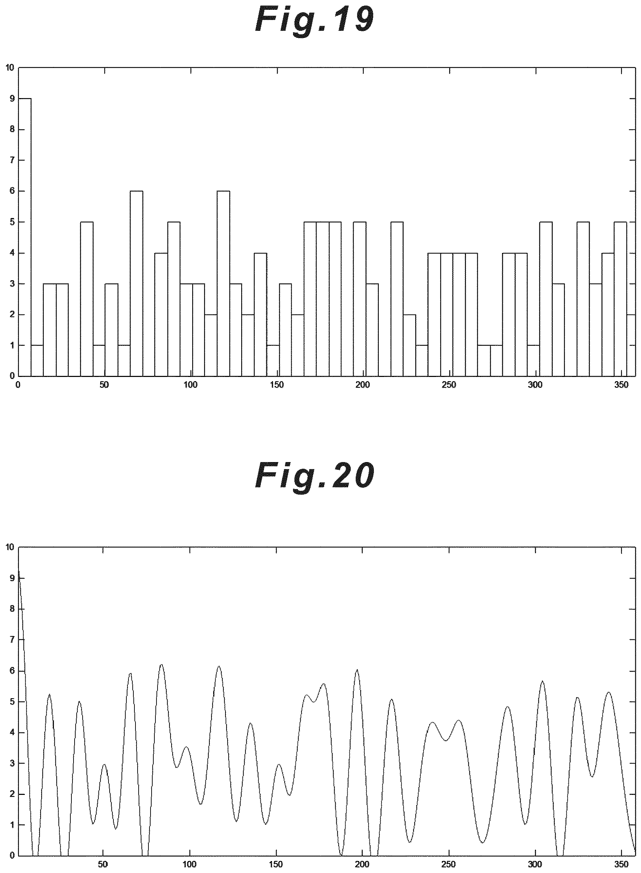

Next, the frequency distribution of timings of which events were extracted at each of a plurality of sites of the brain are acquired. Fig. 19 is a histogram illustrating the frequency distribution of the timings of events extracted in Channels 1 to 6 shown in Fig. 18. Fig. 20 is a smoothed graph of the histogram shown in Fig. 19. In Figs. 19 and 20, the horizontal axis indicates the data numbers of the feature amounts, and corresponds to the time axis.

(5) Determination

Subsequently, it is determined whether a CSD occurred based on the frequency distribution of the timing of the occurrence of an event.

As mentioned above, CSD is a phenomenon where depolarization of neurons and glial cells in brain grey matter propagates slowly. Accordingly, when a CSD occurs, a negative DC shift is observed in different sites of the brain accompanied by a time difference. Thus, by analyzing the frequency distribution of timings of negative DC shifts observed in a plurality of sites of the brain, it can be determined whether an observed negative DC shift is caused by a CSD.

Specifically, it can be determined in the frequency distribution that a CSD occurred in the vicinity of a timing where a peak of a predetermined threshold Th1 or higher was seen. However, if a peak in the frequency distribution exceeds a predetermined threshold Th2 (Th2 > Th1); namely, if negative DC shifts are nearly simultaneously observed in a plurality of sites (channels 1 to 6), it is considered that these negative DC shifts are not caused by a CSD. Accordingly, in the frequency distribution, it is more preferable to determine the results in which peaks exceeding threshold Th2 were removed from among the peaks exceeding threshold Th1, as a timing of a CSD occurrence.

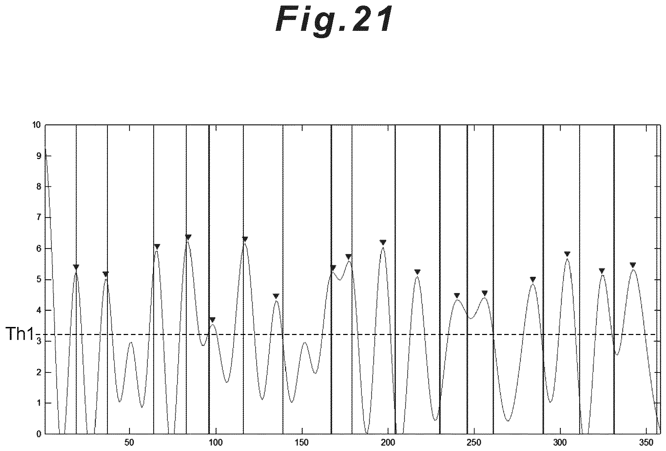

Fig. 21 is a graph illustrating the timings of CSD occurrences extracted from the graph shown in Fig. 20. In Fig. 21, the horizontal axis indicates the data numbers of the feature amounts, and corresponds to the time axis. The horizontal dashed line shown in Fig. 21 indicates a threshold (Th), and the marks '▼' indicate points determined as CSDs. Moreover, the vertical solid lines shown in Fig. 21 indicate the starting points of the CSDs extracted manually by the present inventors. As shown in Fig. 21, it is understood that the timings determined as CSDs in the present example are in the vicinity of the timings of the CSDs extracted manually by the present inventors.

As explained above, according to the present embodiment, a negative DC shift can be quickly and automatically detected by setting windows at predetermined time intervals on the waveform data of the direct current component of the EEG, calculating a feature amount for each of the windows, and performing threshold processing on these feature amounts. Accordingly, the occurrence of a CSD can be quickly sensed based on a negative DC shift thereby detected.

Moreover, according to the present embodiment, a negative DC shift can be precisely extracted by utilizing permutation entropy or power density as feature amounts. Accordingly, it can be precisely sensed whether a CSD occurred based on the timing of a negative DC shift extracted by utilizing such feature amounts.

Moreover, according to the present embodiment, it can be automatically and accurately determined whether a CSD occurred, based on the frequency distribution of the timing of the negative DC shift detected from the biological information in a plurality of sites of the brain.

(Second Example)

In the aforementioned first example, a negative DC shift was detected by using the waveform data of the direct current component of an EEG, and the occurrence of a CSD was sensed. However, the data values of biological information such as hemoglobin concentration measured in accordance with the NIRS principle, cerebral blood flow measured in accordance with the laser Doppler principle or brain temperature also temporarily change when a CSD occurs, similarly to the direct current component of an EEG. Thus, an example is explained in the second example, in which a CSD is sensed by utilizing, in addition to a direct current component of an EEG, brain temperature and hemoglobin concentration as biological information.

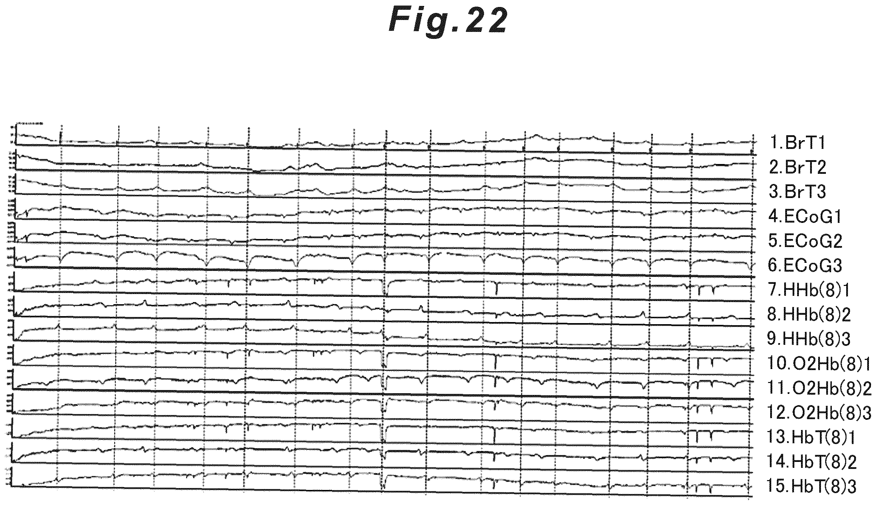

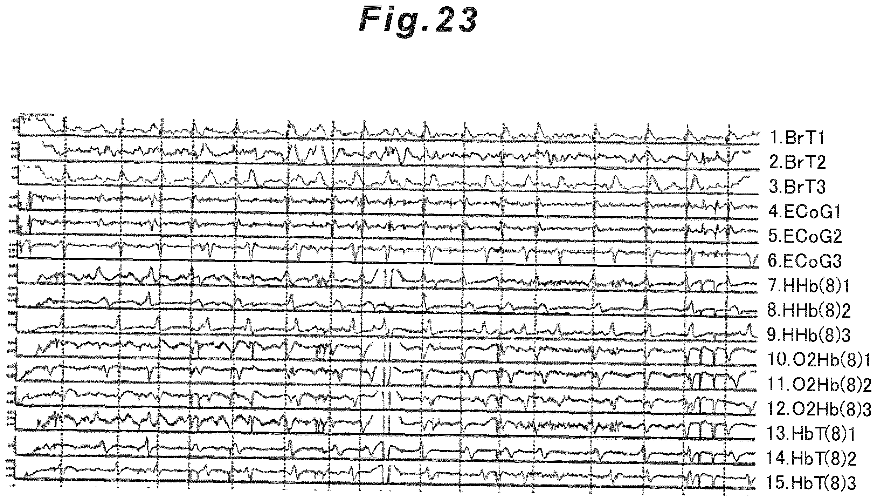

Fig. 22 is a graph illustrating waveforms of brain temperatures (BrT), direct current components of an electrocorticogram (EcoG), and hemoglobin concentrations (de-oxygenated hemoglobin concentration: HHb, oxygenated hemoglobin concentration: O2Hb, total hemoglobin concentration: HbT) measured across three channels. Moreover, Fig. 23 is a graph illustrating waveforms obtained by subjecting the waveforms shown in Fig. 22 to high-pass filtering. Of these, the cutoff frequency was 0.0025 Hz for the direct current components of the ECoG, and the cutoff frequency was 0.001 Hz for the brain temperature and each type of hemoglobin concentration. The dashed lines shown in Fig. 22 and Fig. 23 indicate the timings of negative DC shift occurrences due to a CSD extracted manually by the present inventors. As shown in Fig. 23, behavior was observed in which the values of the brain temperatures and hemoglobin concentrations in each channel also temporarily changed in the vicinity of the timings of the negative DC shifts, similarly to that of the direct current component of the ECoG. In the second example, feature amounts were calculated by setting windows on the waveform data of these brain temperatures, direct current components of the ECoG, and each type of hemoglobin concentration. Permutation entropy was used as a feature amount. Then, events due to CSDs (negative DC shifts etc.) were extracted from the waveform data by subjecting the calculated feature amounts to threshold processing.

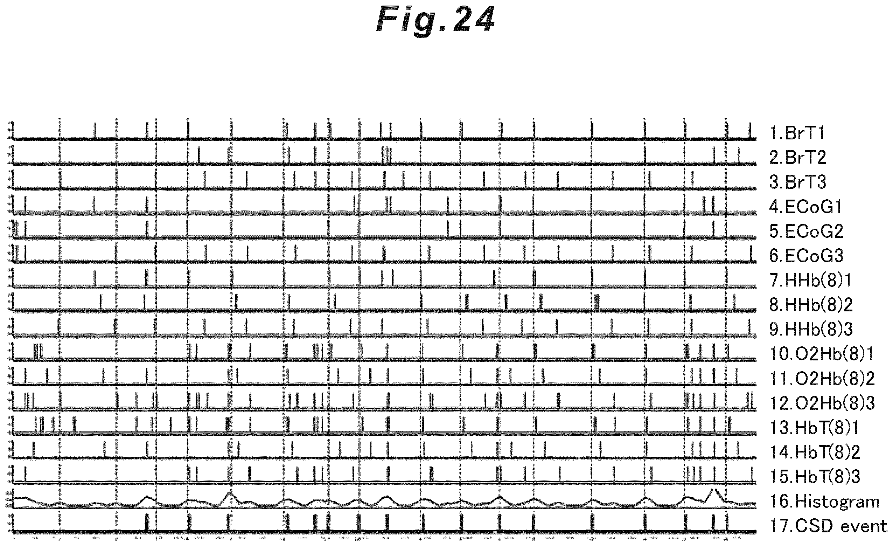

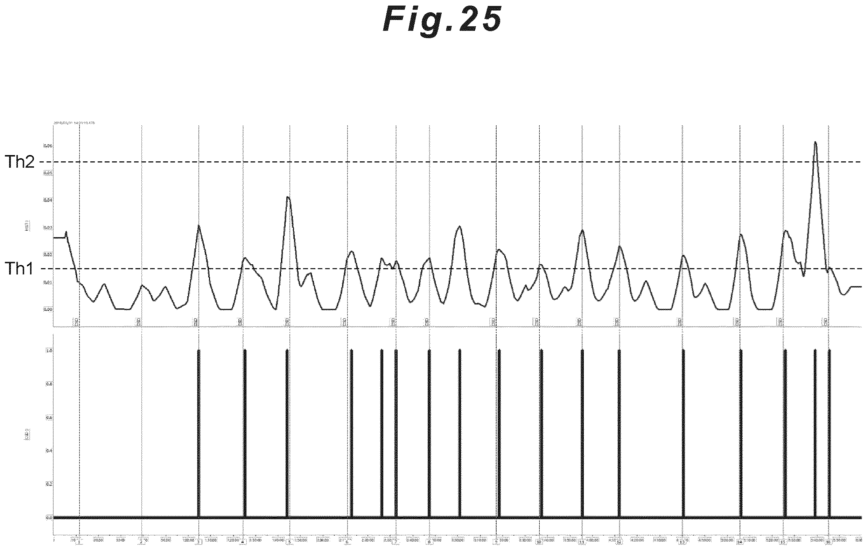

Fig. 24 is a graph illustrating the timings of events extracted based on the waveforms shown in Fig. 23 (channels 1 to 15), a frequency distribution of the extracted timings (channel 16), and the timings of CSD occurrences determined based on the frequency distribution (channel 17). Fig. 25 is an enlarged graph of the frequency distribution (channel 16) and the timings of CSD occurrences shown in Fig. 24.

The frequency distributions shown in Figs. 24 and 25 were obtained by adding all the timings shown in channels 1 to 15 of Fig. 24. Moreover, as shown in Fig. 25, from among the peaks exceeding the threshold Th1 in the frequency distribution, the peaks not exceeding threshold Th2 were sensed as the timings of CSD occurrences. The dashed lines shown in Figs. 24 and 25 indicate the timings of CSD occurrences extracted manually by the present inventors.

As shown in Fig. 25, it is understood that the timings of CSD occurrences sensed based on waveform data of brain temperature, electrocorticogram and hemoglobin concentration substantially match the timings of CSD occurrences extracted manually by the present inventors.

According to the second example, in addition to an electrocorticogram, the number of data can be increased by utilizing brain temperature and hemoglobin concentration, as biological information. Thus, the accuracy of sensing a CSD can be improved.

In the second example, data of an electrocorticogram, brain temperature, and hemoglobin concentration were used as biological information. However, sensing of CSD may also take place by a variety of biological information such as only brain temperature, only hemoglobin concentration, only cerebral blood flow, or by combinations thereof such as by a combination of brain temperature and hemoglobin concentration, or by a combination of brain temperature and cerebral blood flow etc. However, it is preferable to suitably select the resolution of data (order) depending on the biological information.

The present invention is not limited to the embodiments explained above, and other various modes may be implemented within the scope which does not deviate from the gist of the present invention. For example, some components may be excluded from all the components shown in the aforementioned embodiments to form the present invention, or components shown in the aforementioned embodiments may be suitably combined to form the present invention.

(Addendum 1)

A cortical spreading depolarization sensing method which includes:

an acquisition step of acquiring waveform data indicating a temporal change in one or more types of biological information including at least any one of a direct current component of an electroencephalogram, hemoglobin concentration in the brain, cerebral blood flow, and brain temperature;

a feature amount calculation step of setting windows set at predetermined time intervals on the waveform data and calculating permutation entropy as a feature amount for each of the windows; and

an event extraction step of extracting a peak in the feature amount calculated in the feature amount calculation step, and extracting a timing of a peak in which a peak value is below a predetermined threshold as the timing of the occurrence of an event in which the value of the waveform data temporarily changes due to cortical spreading depolarization.

(Addendum 2)

The cortical spreading depolarization sensing method described in Addendum 1, in which the windows each have a width of longer than 0.5 minutes and shorter than 10 minutes.

(Addendum 3)

The cortical spreading depolarization sensing method described in Addenda 1 or 2, in which, the feature amount calculation step includes calculating the feature amount for data obtained by subjecting the waveform data to filtering process to cut off frequency components less than or equal to a predetermined frequency.

(Addendum 4)

A sensing system provided with:

a waveform data acquisition part configured to acquire waveform data indicating a temporal change in one or more types of biological information including at least any one of a direct current component of an electroencephalogram, hemoglobin concentration in the brain, cerebral blood flow, and brain temperature;
a feature amount calculation part to set windows set at predetermined time intervals on the waveform data and calculate permutation entropy as a feature amount for each of the windows; and
an event extraction part to extract a peak in a feature amount calculated in the feature amount calculation part, and extract a timing of a peak in which a peak value is below a predetermined threshold as the timing of the occurrence of an event in which the value of the waveform data temporarily changes due to cortical spreading depolarization.

(Addendum 5)

The sensing system described in Addendum 4, further provided with:

a subdural sensor to be placed in the subdural space and capable of outputting signals indicating biological information concerning the brain; and
a signal processing part capable of generating digital waveform data by subjecting signals output from the subdural sensor to a predetermined signal processing,
where the waveform data acquisition part acquires the waveform data from the signal processing part; and
where the subdural sensor has:

a substrate formed from a flexible material and placed so as to be in contact with a brain surface, and
a plurality of sensor units each mounted in a plurality of locations of the substrate on the side that is in contact with a brain surface, each sensor unit including at least any one of electrodes for measuring an electrocorticogram, a measurement part for measuring a hemoglobin concentration or a tissue oxygen saturation by near-infrared spectroscopy, a blood flowmeter for measuring a cerebral blood flow in accordance with the laser Doppler principle, and a temperature sensor.

(Addendum 6)

A program for executing on a computer:

an acquisition step of acquiring waveform data indicating a temporal change in one or more types of biological information including at least any one of a direct current component of an electroencephalogram, hemoglobin concentration in the brain, cerebral blood flow, and brain temperature;
a feature amount calculation step of setting windows set at predetermined time intervals on the waveform data and calculating permutation entropy as a feature amount for each of the windows;
an event extraction step of extracting a peak in the feature amount calculated in the feature amount calculation step, and extracting a timing of a peak in which a peak value is below a predetermined threshold as the timing of the occurrence of an event in which the value of the waveform data temporarily changes due to cortical spreading depolarization.

DESCRIPTION OF REFERENCE NUMBERS

**[0105]** 1 ... Sensing system, 10 ... Subdural sensor, 20 ... Signal processing part, 30 ... Information processing device, 31 ... External interface, 32 ... Storage part, 33 ... Processor, 34 ... Operation input part, 35 ... Display device, 100 ... Substrate, 101 ... Sensor region, 102 ... Wiring region, 104 ... Front surface, 105 ... Back surface, 110 ... Sensor unit, 111 ... Light emitting element, 112 ... Light receiving element, 113 ... Electrode, 120 ... Temperature sensor, 130 ... Intracranial pressure sensor, 201 ... Scalp, 202 ... Cranium, 203 ... Burr hole, 204 ... Dura mater, 205 ... Subdural space, 206 ... Brain surface, 321 ... Program storage part, 322 ... Waveform data storage part, 323 ... Threshold storage part, 331 ... Waveform data acquisition part, 332 ... Feature amount calculation part, 333 ... Event extraction part, 334 ... Statistical processing part, 335 ... Determination part

**Claims**

1. A cortical spreading depolarization sensing method, comprising:

   an acquisition step of acquiring, for each of a plurality of sites of a brain, waveform data indicating a temporal change in one or more types of biological information including at least any one of a direct current component of an electroencephalogram, hemoglobin concentration in the brain, cerebral blood flow, and brain temperature;
   a feature amount calculation step of setting windows set at predetermined time intervals on the waveform data and calculating a predetermined feature amount for each of the windows;
   an event extraction step of performing threshold processing on the feature amounts calculated in the feature amount calculation step to extract a timing of the occurrence of an event in which a value of the waveform data temporarily changes due to cortical spreading depolarization;
   a statistical processing step of acquiring a frequency distribution of the timings extracted based on the plurality of waveform data acquired in relation to a plurality of sites of the brain; and
   a determination step of determining whether cortical spreading depolarization has occurred based on the frequency distribution.

2. The cortical spreading depolarization sensing method according to claim 1, wherein the determination step includes determining cortical spreading depolarization to have occurred when a peak value of frequency in the frequency distribution is in a predetermined range.

3. The cortical spreading depolarization sensing method according to claim 1 or 2, wherein the determination step includes determining timings of peaks exceeding a first threshold in the frequency distribution except for timings of peaks exceeding a second threshold that is larger than the first threshold as timings of cortical spreading depolarization occurrence.

4. The cortical spreading depolarization sensing method according to any one of claims 1 to 3,

   wherein the acquisition step includes acquiring waveform data relating to a plurality of types of biological information; and
   wherein the statistical processing step includes obtaining the frequency distribution based on the timings extracted on the basis of the plurality of types of biological information.

5. The cortical spreading depolarization sensing method according to any one of claims 1 to 4,

   wherein the feature amount is permutation entropy, and
   wherein the event extraction step includes extracting a negative peak below a threshold.

6. The cortical spreading depolarization sensing method according to any one of claims 1 to 4,

   wherein the feature amount is power density, and
   wherein the event extraction step includes extracting a positive peak exceeding a threshold.

7. The cortical spreading depolarization sensing method according to any one of claims 1 to 6, wherein the windows each have a width of longer than 0.5 minutes and shorter than 10 minutes.

8. The cortical spreading depolarization sensing method according to any one of claims 1 to 7, wherein the feature amount calculation step includes calculating the feature amount for data obtained by subjecting the waveform data to filtering process to cut off frequency components less than or equal to a predetermined frequency.

9. A sensing system comprising:
a waveform data acquisition part configured to acquire, for each of a plurality of sites of a brain, waveform data indicating a temporal change in one or more types of biological information including at least any one of a direct current component of an electroencephalogram, hemoglobin concentration in the brain, cerebral blood flow, and brain temperature;

a feature amount calculation part configured to set windows set at predetermined time intervals on the waveform data and calculate a predetermined feature amount for each of the windows;
an event extraction part configured to perform threshold processing on the feature amounts calculated in the feature amount calculation part to extract a timing of the occurrence of an event in which a value of the waveform data temporarily changes due to cortical spreading depolarization;
a statistical processing part to acquire a frequency distribution of the timings extracted based on the plurality of waveform data acquired in relation to a plurality of sites of the brain; and
a determination part configured to determine whether cortical spreading depolarization has occurred based on the frequency distribution.

10. The sensing system according to claim 9, further comprising:

a subdural sensor to be placed in the subdural space and capable of outputting signals indicating biological information concerning the brain; and
a signal processing part capable of generating digital waveform data by subjecting signals output from the subdural sensor to a predetermined signal processing,
wherein the waveform data acquisition part acquires the waveform data from the signal processing part; and
wherein the subdural sensor comprises:

a substrate formed from a flexible material and placed so as to be in contact with a brain surface, and
a plurality of sensor units each mounted in a plurality of locations of the substrate on the side that is in contact with a brain surface, each sensor unit comprising at least any one of electrodes for measuring an electrocorticogram, a measurement part for measuring a hemoglobin concentration or a tissue oxygen saturation by near-infrared spectroscopy, a blood flowmeter for measuring a cerebral blood flow in accordance with the laser Doppler principle, and a temperature sensor.

11. A program for executing on a computer:

an acquisition step of acquiring, for each of a plurality of sites of a brain, waveform data indicating a temporal change in one or more types of biological information including at least any one of a direct current component of an electroencephalogram, hemoglobin concentration in the brain, cerebral blood flow, and brain temperature;
a feature amount calculation step of setting windows set at predetermined time intervals on the waveform data and calculating a predetermined feature amount for each of the windows;
an event extraction step of performing threshold processing on the feature amounts calculated in the feature amount calculation step to extract a timing of the occurrence of an event in which a value of the waveform data temporarily changes due to cortical spreading depolarization;
a statistical processing step of acquiring a frequency distribution of the timings extracted based on the plurality of waveform data acquired in relation to a plurality of sites of the brain; and
a determination step of determining whether cortical spreading depolarization has occurred based on the frequency distribution.

# *Fig.1*

```
        ┌─────────┐
        │  START  │
        └────┬────┘
             │
             ▼
  ┌────────────────────┐
  │      ACQUIRE       │──── S10
  │   WAVEFORM DATA    │
  └─────────┬──────────┘
            │
            ▼
  ┌────────────────────┐
  │     CALCULATE      │──── S11
  │  FEATURE AMOUNTS   │
  └─────────┬──────────┘
            │
            ▼
  ┌────────────────────┐
  │ EXTRACT TIMING OF  │
  │   THE OCCURRENCE   │──── S12
  │     OF EVENTS      │
  └─────────┬──────────┘
            │
            ▼
  ┌────────────────────┐
  │ ACQUIRE FREQUENCY  │──── S13
  │    DISTRIBUTION    │
  └─────────┬──────────┘
            │
            ▼
  ┌────────────────────┐
  │   DETERMINATION    │──── S14
  └─────────┬──────────┘
            │
            ▼
        ┌─────────┐
        │   END   │
        └─────────┘
```

# Fig.2

<u>1</u>

30

**INFORMATION PROCESSING DEVICE**

31 — EXTERNAL INTERFACE

33 — **PROCESSOR**

331 — WAVEFORM DATA ACQUISITION PART

332 — FEATURE AMOUNT CALCULATION PART

333 — EVENT EXTRACTION PART

334 — STATISTICAL PROCESSING PART

335 — DETERMINATION PART

32 — **STORAGE PART**

321 — PROGRAM STORAGE PART

322 — WAVEFORM DATA STORAGE PART

323 — THRESHOLD STORAGE PART

10 — SUBDURAL SENSOR

20 — SIGNAL PROCESSING PART

34 — OPERATING INPUT PART

35 — DISPLAY DEVICE

# Fig.3

# Fig.4

# Fig.5

# Fig.6

# Fig.7

Permutation Entropy

# Fig.8

Power Density

# Fig.9

Amplitude Modulation

# Fig.10

# Fig.11

RMS Frequency

# Fig.12

# Fig.13

# Fig.14

# Fig.15

# Fig.16

# Fig.17

## Fig.18

*Fig.19*

*Fig.20*

# Fig.21

*Fig.22*

1.BrT1
2.BrT2
3.BrT3
4.ECoG1
5.ECoG2
6.ECoG3
7.HHb(8)1
8.HHb(8)2
9.HHb(8)3
10.O2Hb(8)1
11.O2Hb(8)2
12.O2Hb(8)3
13.HbT(8)1
14.HbT(8)2
15.HbT(8)3

# Fig.23

1.BrT1
2.BrT2
3.BrT3
4.ECoG1
5.ECoG2
6.ECoG3
7.HHb(8)1
8.HHb(8)2
9.HHb(8)3
10.O2Hb(8)1
11.O2Hb(8)2
12.O2Hb(8)3
13.HbT(8)1
14.HbT(8)2
15.HbT(8)3

Fig.24

**Fig.25**

## INTERNATIONAL SEARCH REPORT

| International application No. |
| --- |
| **PCT/JP2022/011730** |

**A. CLASSIFICATION OF SUBJECT MATTER**

*A61B 5/01*(2006.01)i; *A61B 5/026*(2006.01)i; *A61B 5/1459*(2006.01)i; *A61B 5/37*(2021.01)i; *A61B 5/372*(2021.01)i
FI:   A61B5/372; A61B5/01 250; A61B5/026 120; A61B5/1459; A61B5/37

According to International Patent Classification (IPC) or to both national classification and IPC

**B. FIELDS SEARCHED**

Minimum documentation searched (classification system followed by classification symbols)

A61B5/01; A61B5/026; A61B5/1459; A61B5/37; A61B5/372

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | US 2016/0143574 A1 (JONES, Stephen Carter) 26 May 2016 (2016-05-26) paragraphs [0020], [0072]-[0074], [0094]-[0095] | 1-11 |
| A | US 2018/0085047 A1 (UNIVERSITY OF CINCINNATI) 29 March 2018 (2018-03-29) paragraph [0032], examples 2-3 | 1-11 |
| A | 岡史朗，外9名, 重症脳損傷患者におけるcortical spreading depolarization 発生時の脳温および脳循環代謝の変化, 第61回日本脳循環代謝学会学術集会 一般演題 口演抄録, 2018 entire text, (OKA, Shiro et al.), non-official translation (Changes in brain temperature and cerebral circulation metabolism during cortical spreading depolarization in patients with severe craniocerebral injury. General Presentation and Oral Abstract of the 61st Annual Meeting of the Japanese Society of Cerebrat Blood Flow and Metabolism.) | 1-11 |
| A | JP 2015-221177 A (UNIV YAMAGUCHI) 10 December 2015 (2015-12-10) claims 1-5, examples | 1-11 |

☐ Further documents are listed in the continuation of Box C.     ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **17 May 2022** | **31 May 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/011730**

| Patent document cited in search report | Publication date (day/month/year) | Patent family member(s) | Publication date (day/month/year) |
|---|---|---|---|
| US 2016/0143574 A1 | 26 May 2016 | WO 2016/085909 A2 paragraphs [0019], [0049]-[0051], [0070]-[0071] EP 3223693 A2 | |
| US 2018/0085047 A1 | 29 March 2018 | WO 2016/160047 A1 p. 9, line 17 to p. 10, line 3, examples 2-3 | |
| JP 2015-221177 A | 10 December 2015 | (Family: none) | |

Form PCT/ISA/210 (patent family annex) (January 2015)

**REFERENCES CITED IN THE DESCRIPTION**

**Patent documents cited in the description**

- JP 2015047452 A
- JP 6296606 B

**Non-patent literature cited in the description**

- **MAHAPATRA et al.** Classification of ictal and interictal EEG using RMS frequency, dominant frequency, root mean instantaneous frequency square and their parameters ratio. *Biomedical Signal Processing and Control,* 2018, vol. 44, 168-180
- **SUGANO HIDENORI et al.** Focal diagnosis of epilepsy using entropy as a feature from interictal EEG. *Japanese Journal of Clinical Neurophysiology,* 2020, vol. 48 (3), 107-112
- **UNAKAFOVA et al.** Efficiently Measuring Complexity on the Basis of Real-World Data. *Entropy,* 2013, vol. 15, 4392-4415
- **YAMAKAWA TOSHITAKA et al.** Implantable Multi-Modality Probe for Subdural Simultaneous Measurement of Electrophysiology, Hemodynamics, and Temperature Distribution. *IEEE TRANSACTIONS ON BIOMEDICAL ENGINEERING,* November 2019, vol. 66 (11), 3204-3211
- **SUGIMOTO KAZUTAKA et al.** Spreading depolarization (CSD) appearing in subarachnoid hemorrhage - Blood flow is reduced when CSD reaches a pathological cerebral cortex. *Japan Medical Journal, Weekly Nihon Iji Shimpo,* 20 February 2016, (4791), 52
- **BANDT et al.** Permutation Entropy: A Natural Complexity Measure for Time Series. *PHYSICAL REVIEW LETTERS,* 29 April 2002, vol. 88 (17), 174102-1, 174102-4